Europäisches Patentamt

(19) European Patent Office

Office européen des brevets

(11) Publication number: **0 102 801**

**B1**

(12) **EUROPEAN PATENT SPECIFICATION**

(45) Date of publication of patent specification: **22.06.88**

(51) Int. Cl.⁴: **C 12 P 1/00** // C12R1/91, A61K35/12

(21) Application number: **83304845.7**

(22) Date of filing: **22.08.83**

(54) An agent for inhibiting proliferation of malignant tumour cells.

(30) Priority: **20.08.82 JP 143340/82**

(43) Date of publication of application:
**14.03.84 Bulletin 84/11**

(45) Publication of the grant of the patent:
**22.06.88 Bulletin 88/25**

(84) Designated Contracting States:
**CH DE FR GB LI NL**

(56) References cited:
**EP-A-0 087 087
DE-A-2 314 076
DE-A-2 600 442
DE-A-2 619 715
FR-A-2 302 747
US-A-4 059 486
US-A-4 228 236**

**BIOLOGICAL ABSTRACTS, vol. 63, 1st March
1977, abstract no. 27753; E. KEDAR et al.: "In
vitro induction of cell-mediated immunity to
murine leukenia cells: I. Optimization of tissue
culture conditions for the generation of
cytotoxic lymphocytes" & J. IMMUNOL.**

(73) Proprietor: **KOKEN LTD.
No. 7, Yonban-cho
Chiyoda-ku Tokyo (JP)**

(73) Proprietor: **Oishi, Tadakatsu
No. 55-1, Nakajima-Akinoyama-cho Fushimi-ku
Kyoto-shi Kyoto-fu (JP)**

(73) Proprietor: **Tajima, Tomoyuki
5-15, Yawata 6-chome
Ichikawa-shi Chiba-ken (JP)**

(73) Proprietor: **Nagasu, Youichiro
No. 9-4, Chuo 3-chome
Yamato-shi Kanagawa-ken (JP)**

(72) Inventor: **Tajima, Tomoyuki
No. 5-15, Yawata 6-chome
Ichikawa-shi Chiba-ken (JP)**

(74) Representative: **Deans, Michael John Percy et al
Lloyd Wise, Tregear & CO. Norman House
105-109 Strand
London WC2R OAE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

(56) References cited:

**METHODS 13(1), 1-19, 1976**

**BIOLOGICAL ABSTRACTS, vol. 69, 1980, abstract no. 72929; M.R. YOUNG et al.: "Selective inhibition of tumor cell migration by culture supernatants derived from normal and lipopolysaccharide activated macrophages" & RES. J. RETICULOENDOTHEL SOC. 27(2), 143-150, 1980**

**J. NATL. CANCER INST. VOL. 56 Nr. 5, May 76, pp. 899-902**

**J. IMMUN. vol. 121, Nr. 1, July 1978 pp. 44-52**

## Description

The present invention relates to agents for inhibiting the proliferation of malignant tumour cells. A number of anti-tumour agents have been previously proposed. They often suffer from serious disadvantages and exhibit many side effects in use in inhibiting the proliferation of malignant tumour cells.

Malignant tumour cells have been subjected to very extensive studies over a long period of time. Among the many lines of research involving such cells, Remacle-Bonnet et al in Journal of Immunology Vol. 121, No. 1, July 1978 pp. 44—52 disclose studies with soluble extracts from human colonic carcinoma demonstrating a non-specific suppressive and cytostatic activity against a wide variety of proliferating cells. The said paper also refers to earlier work of Houck et al J. Natl. Cancer Inst. Vol. 56, No. 5, May 1976 pp. 899—902 providing evidence for a colon chalone in medium ultrafiltrates from the culture fluid in which the cells had previously grown. The inhibitory activity was concentrated in the molecular weight range between 10.000 and 5.0000 daltons. The factor concerned appeared to be present both in normal and malignant colon mucosa.

European Patent Application No. EP—A—87087 filed 11th February, 1983 and published 31st August 1983, discloses a method said to produce anti-tumour substances by culturing tumour cells in the presence of an inducer such as liposaccharide, lipin A, etc. Moreover, the resultant substances are said to show a molecular weight of 40,000 to 50,000.

In contrast, according to a first aspect of the present invention, there is provided an agent for inhibiting proliferation of malignant tumour cells, characterised in that said agent is obtained by the steps of: culturing in a medium supplemented with serum selected from new born calf serum, cattle serum, calf serum, cattle embryo serum and like serum derived from other species human malignant cells selected from human renal carcinoma cells, human gastric carcinoma cells, human lung carcinoma cells, carcinoma cells from the human oral cavity or human myogenic sarcoma cells; washing once to remove the serum; transferring the cultured human malignant tumour cells to a separation medium without serum supplement; incubating the thus transferred cells; and removing from said separation medium both said malignant tumour cells and molecules having a molecular weight of not less than 1000 daltons.

In a second and alternative aspect of the present invention, we provide a process for the production of an agent for inhibiting proliferation of malignant tumour cells, the process being characterised in that it comprises the steps of: culturing in a medium supplemented with serum selected from new born calf serum, cattle serum, calf serum, cattle embryo serum and like serum derived from other species human malignant cells selected from human renal carcinoma cells, human gastric carcinoma cells, human lung carcinoma cells, carcinoma cells from the human oral cavity or human myogenic sarcoma cells; washing once to remove the serum; transferring the cultured human malignant tumour cells to a separation medium without serum supplement; incubating the thus transferred cells; and removing from said separation medium both said malignant tumour cells and molecules having a molecular weight of not less than 1000 daltons.

The inhibitors produced by the process of the present invention have a molecular weight of 1000 daltons or less and are plainly to be distinguished from any inhibitor produced by the method of European Patent Application EP—A—87087.

As explained in detail below with reference to specific examples, inhibitors so produced can inhibit the proliferation of tumour cells and have a cytolytic effect thereon. Our experimental work shows that the inhibitor agents produced having a relatively low molecular weight may pass through a filter such as a YM2 or UM05 filter manufactured by Amicon, which is capable of passing a substance having a molecular weight of 500 or 1000 daltons therethrough. The inhibitor substance may show some inhibitive effect on the growth of normal cells; however, its ability to inhibit the proliferation of malignant tumour cells is substantially greater than its ability to inhibit the proliferation of normal cells. Morever, we have not observed any cytolytic effect against normal cells. We therefore conclude that our inhibitor agents will show few of the side effects which often appear with conventional anti-tumour agents.

The culture is desirably conducted in a growth medium comprising a synthetic medium supplement with a 5% or more new born calf serum. In a preferred example, the synthetic medium is Basal Medium Eagle and is supplemented with 10% new born calf serum. In a second preferred example, the synthetic medium is "RPMI1646", and is supplemented with 5% new born calf serum.

The separating medium is suitably a synthetic medium and in the preferred example is Basal Medium Eagle.

The step of removal of the malignant tumour cells is suitably achieved by using a molecular sieve capable of filtering a substance having a molecular weight of not less than 500 daltons.

The culture step may be conducted as a co-culture with normal fibroblasts. The normal fibroblasts are preferably normal human diploid skin fibroblasts.

The invention is hereinafter more particularly described with reference to a number of experimental examples and with reference to the accompaning drawings, in which:—

Fig. 1 is a graph illustrating the proliferation of an established cell line of human renal carcinoma (hereafter "HRC") in each of two media;

Figs. 2A and 2B respectively illustrate the proliferation in each of two media of normal human diploid skin fibroblasts explanted from the trunk skin of a normal male aged 63 (hereafter "NAS 63") and an established cell line from a human gastric carcinoma (hereafter "MK");

Fig. 3 is a graph illustrating the proliferation in each of two media of an established cell line originating from a human lung carcinoma (hereafter "PC-1");

Fig. 4 is a graph illustrating the proliferation in each of two media of an established cell line derived from a carcinoma of the human oral cavity (hereafter "KB");

Fig. 5 is a graph illustrating the proliferation in each of two media of an established cell line derived from human myogenic sarcoma (hereafter "HMS");

Fig. 6 is a graph illustrating the dosage responses for HRC for various mixing ratios of a cultured medium derived in accordance with the present invention from HRC with a fresh medium;

Fig. 7 is a graph generally similar to Fig. 6 showing the dosage responses of normal human diploid skin fibroblasts NAS 63 for the same media as employed in Fig. 6;

Fig. 8 is a graph illustrating the proliferation of carcinoma cells in each of various media;

Fig. 9 is a graph similar to Fig. 8 illustrating the proliferation of normal cells in the same media;

Fig. 10 is a graph illustrating the proliferation of carcinoma cells in each of three different media;

Fig. 11 is a graph illustrating the proliferation of normal cells and of carcinoma cells in media comprising various ratios of a co-cultured medium to fresh normal medium;

Fig. 12 is a graph illustrating the proliferation of carcinoma cells of different types in media comprising various ratios of a co-cultured medium to fresh normal medium;

Fig. 13 is a graph illustrating the proliferation of carcinoma cells in a medium supplemented with material obtained by dialysis of a co-cultured medium in a separation medium.

Fig. 14 is a graph illustrating the proliferation of tumour cells in various media inoculated with agents produced in accordance with the present invention employing different number of tumour cells; and

Fig. 15 is a graph showing the proliferation of KB cells in each of a variety of media inoculated with agents produced according to the present invention employing different numbers of HRC cells.

The invention is illustrated by way of example with reference to the following experiments:

Experiment 1

1) Cells used as experimental materials

We employed for this experiment established cell lines separated from five different human tumours, namely: HRC derived from a human renal carcinoma; MK from a human gastric carcinoma; PC-1 from a human lung carcinoma; KB from a carcinoma of the human oral cavity; and HMS from a human myogenic sarcoma.

2) Culture

As growth media we employed Basal Medium Eagle (hereafter "MBE") supplemented with 10% new born calf serum and RPMI1646 supplemented with 5% new born calf serum. As a separating medium we used MBE without serum supplement.

The culture may be carried out by proliferating human malignant tumour cells in a growth medium to a degree at which the growth medium·is saturated with the human malignant tumour cells, and the medium is washed once to remove the serum. These cells may then be incubated in their entirety with a separating medium at 37°C for 3 or 4 days; alternatively a known number of cells collected from the cells proliferated hereinabove may be inoculated into a separating medium and then incubated at 37°C for 3 or 4 days. This is followed by collection of the incubated medium.

3) Partial purification

The cultured medium is then filtered through molecular sieves capable of passing therethrough substances with molecular weights of 10,000, 1,000 and 500 daltons, respectively, and the filtrate is collected.

4) Assay of inhibitor to human malignant tumour cells

Cells used for the assay are the five different cell lines mentioned above as experimental materials, and normal human diploid skin fibroblasts explanted from the trunk skin of a normal male aged 63 (NAS 63).

The proliferation of cells is measured with reference to a growth curve and a dose response curve.

4a) Growth curve

A given number of cells is inoculated into a culture medium which is then incubated with a growth medium which has been prepared by adding to a prefiltered separation medium the same composition and amount of glucose, amino acids and vitamins as in BME, and then supplementing the medium with 10% new born calf serum. The number of cells is counted daily or after a given period of culture.

4b) Dose response curve

A cultured medium is mixed with a corresponding fresh medium in various amounts and nutritive materials added thereto in a similar manner as in the preparation for the growth curves. The number of cells is counted after a predetermined period of incubation.

5) Experimental results

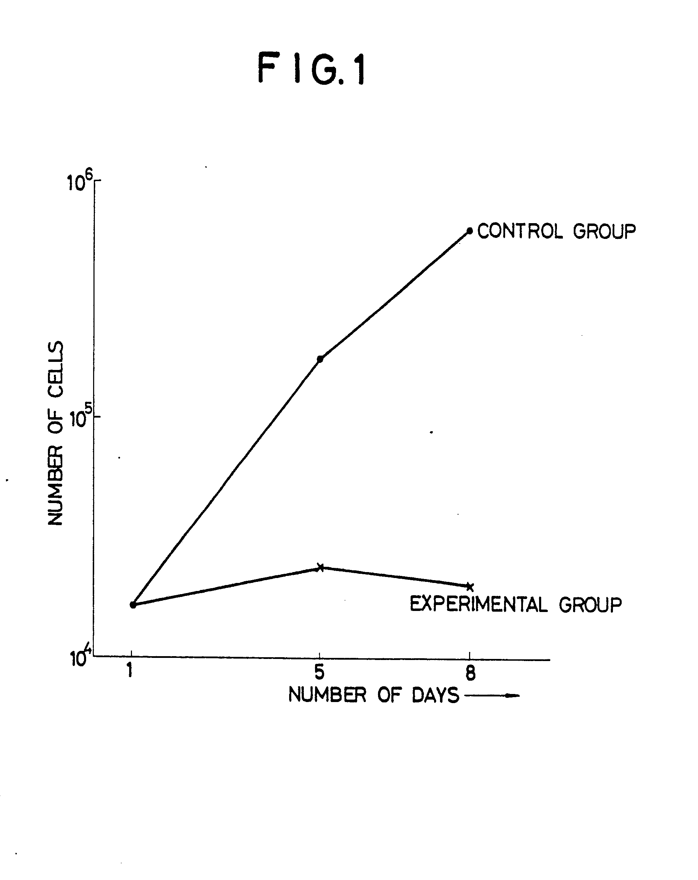

Figs. 1 to 5, respectively, show growth curves illustrating the proliferation of cells in various media including the cultured medium.

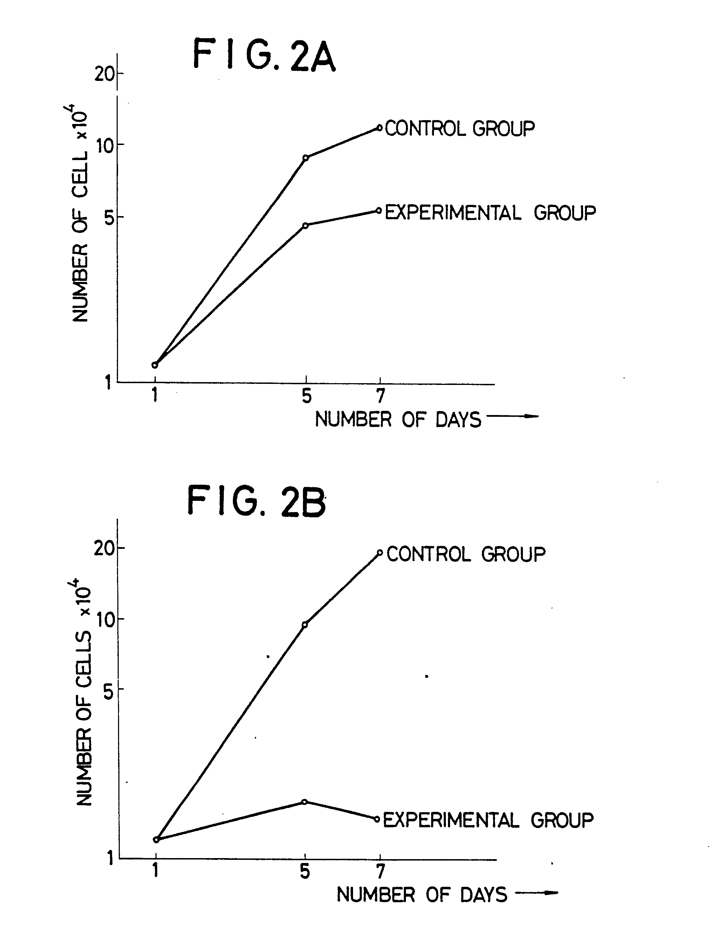

These experiments involve the use of a medium prepared by adding glucose, amino acids, vitamins and 10% serum as nutritive sources to an experimental medium obtained by ultrafiltration through an Amicon YM5 filter (M.W. $10^3$) of a cultured medium after the incubation of the respective malignant tumour cells. Fresh BME supplemented with the same nutritive sources is used as a control. The initial concentration of cells is $1 \times 10^4$ cells. In the first experiment, the results of which are shown in Fig. 1, a disk having a diameter of 35 mm is used, and in subsequent experiments the results of which are shown in Figs. 2 to 5 respectively, a disk having a diameter of 15 mm is used.

Fig. 1 shows the results obtained in an experiment on the proliferation of HRC. It is apparent from the experimental results that the "experimental group" containing an agent in accordance with the present invention obtained by separating medium incubated with HRC inhibits the proliferation of such cells, while the proliferation of such cells is recognized in the "control group".

Fig. 2A illustrates the proliferation of normal human diploid skin fibroblasts NAS 63, and Fig. 2B illustrated the same for MK. In each "experimental group", a medium is employed containing an agent according to the present invention obtained by separating medium incubated with MK. The growth curves shown in Figs. 2A and 2B, respectively, show that the "experimental groups" have an inhibitive effect on cell proliferation as compared to the control, but that the inhibitive effect is much less for the normal cells than for MK.

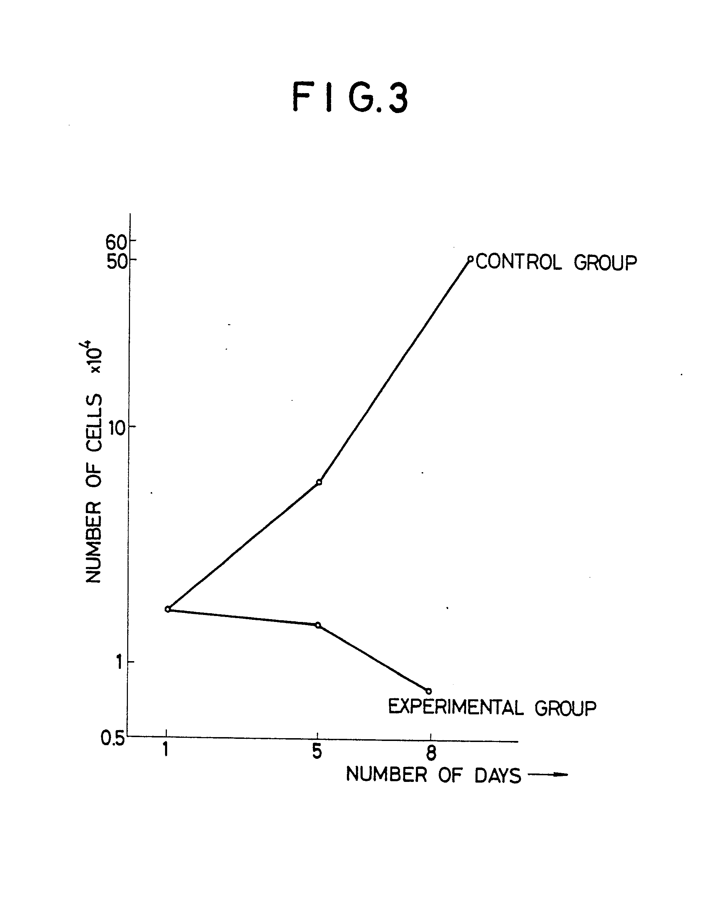

Fig. 3 illustrates the proliferation of PC-1. The experimental results show a decrease in the number of cells in the "experimental group" containing an agent in accordance with the present invention obtained by separating medium incubated with PC-1, and demonstrates the cytolytic properties of the agent concerned.

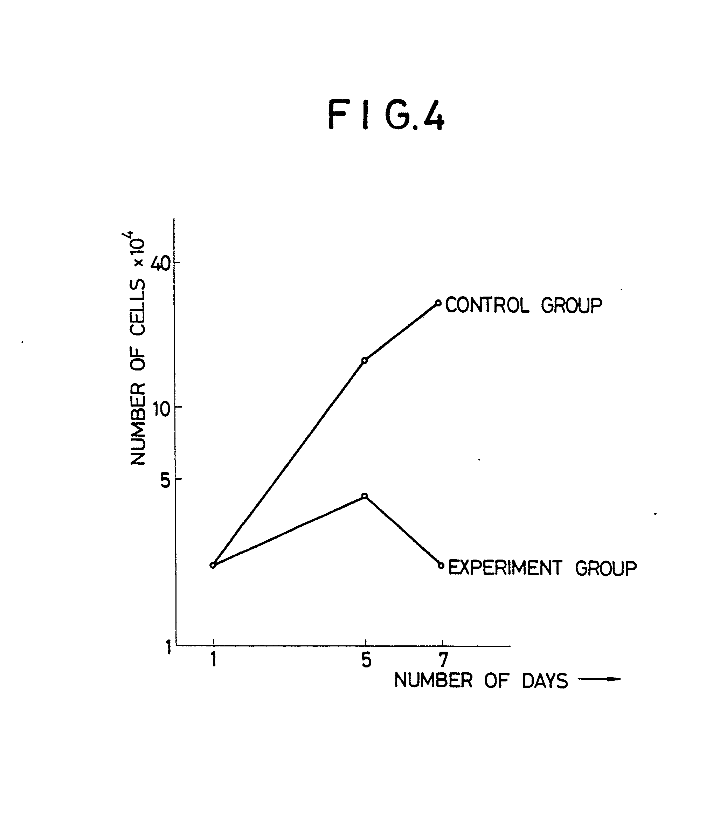

Fig. 4 illustrates the proliferation of KB. In the "experimental group" containing an agent in accordance with the present invention obtained by separating medium incubated with KB, we found that the cells proliferate temporarily and then decline in number.

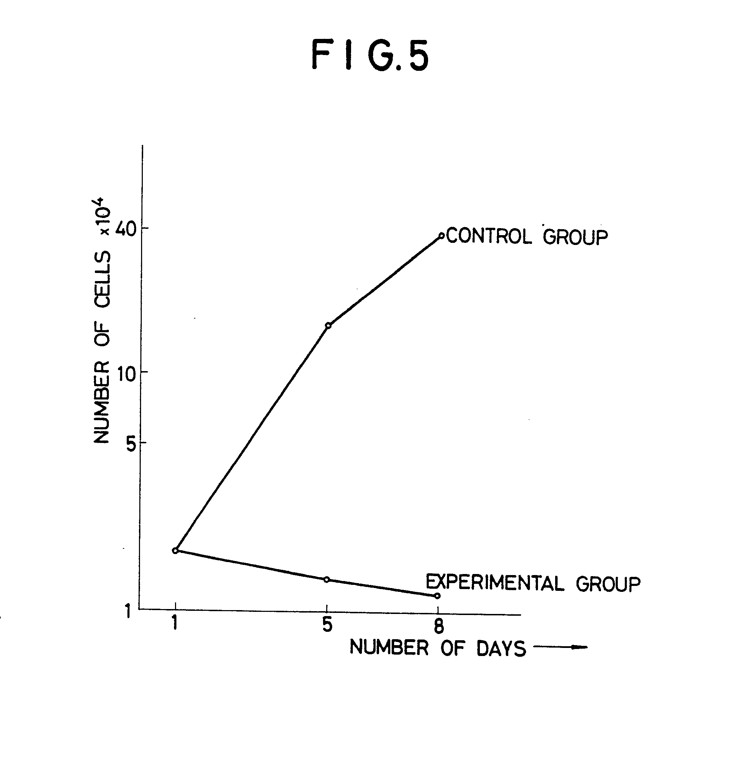

Fig. 5 illustrates the proliferation of HMS. In the "experimental group" containing an agent in accordance with the present invention obtained by separating medium incubated with HMS, we found that the number of cells decreases, demonstrating the cytolytic effect.

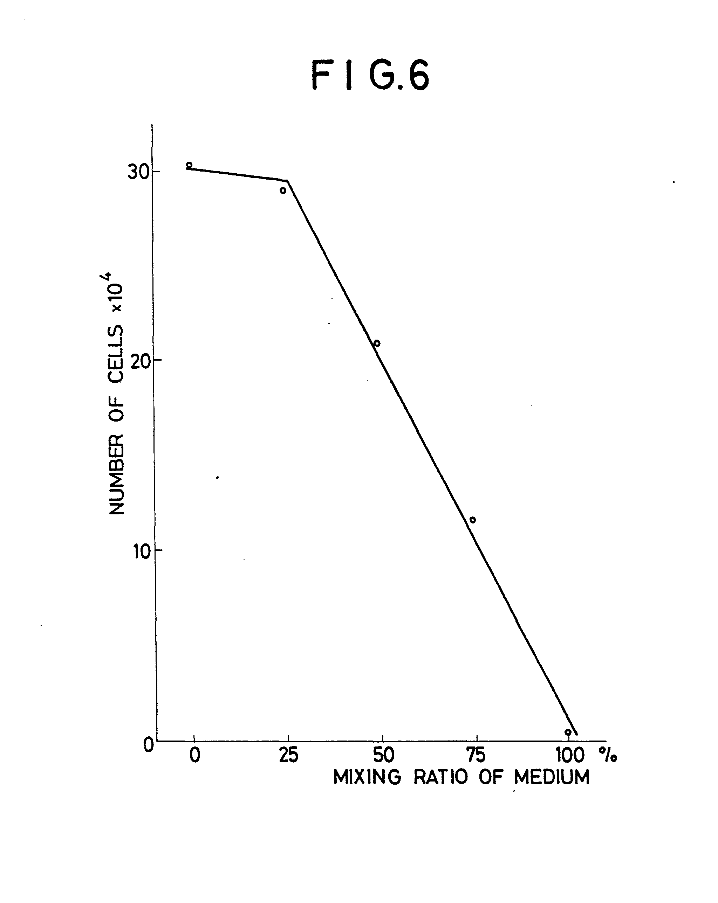

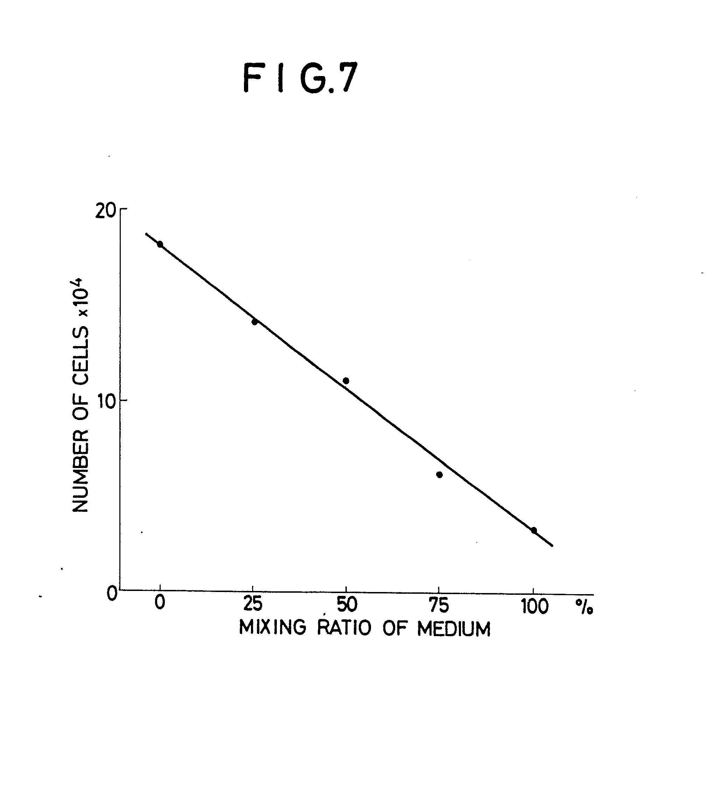

Figs. 6 and 7 show the results of experiments conducted to determine dose responses for various ratios of fresh medium to medium incubated with HRC and thus comprising an agent according to the present invention. Fig. 6 illustrates the dose response curve for HRC, and Fig. 7 illustrates that of normal human diploid skin fibroblasts NAS 63. In each experiment, after incubation, the culture medium used is passed through an Amicon YM2 filter capable of filtering substances with a molecular weight of 1000 daltons. Each ratio (expressed as a percentage) is the ratio of the incubated medium to the sum of the incubated medium and fresh medium. These figures indicate that the inhibitive effects on proliferation of HRC are stronger than those in respect of normal cells in the incubated medium.

Table 1 shows a comparison of the effects on cell proliferation between fractions of different molecular weights obtained by ultrafiltration. In this comparison, normal human diploid skin fibroblasts NAS 63 and HRC are employed.

In Table 1, symbols ①, ② and ③ respectively, indicate the number of each group. Groups ① and ② contain fractions having molecular weights of $10^3$ daltons or lower and show inhibitive and cytolytic effects against established human renal carcinoma cell lines. These fractions are shown to have some inhibitive influence on normal cells, too, but no cytolytic effect against normal cells can be seen by morphological observation.

TABLE 1

| Fractions | NAS 63 | | HRC | |
|---|---|---|---|---|
| | Cell $(\times 10^4)$ numbers | % | Cell $(\times 10^4)$ numbers | % |
| Control | 6.30 | 100 | 21.14 | 100 |
| ①$\leq 10^3$ | 1.58 | 25 | 0.33 | 1.6 |
| ②$\leq 10^3$ | 1.72 | 27 | 0.31 | 1.5 |
| $10^3 \leq$③$< 10^4$ | 3.78 | 60 | 17.87 | 85 |

Experiment 2

1) Cells used as experimental materials

We employed for this experiment normal human diploid skin fibroblasts explanted from the trunk skin of males aged 53 and 63, respectively, and an established cell line from a human renal carcinoma (HRC).

5

2) Culture

As a growth medium we employed Basal Medium Eagle (BME) supplemented with 10% new born calf serum.

The conditions for incubation were either a closed system or a system open to the air and containing 5% $CO_2$ and having a 100% humidity. Incubation was carried out at 37°C.

A combined or co-culture is conducted by first incubating human fibroblasts in the medium set forth hereinabove. When the culture vessel is fully covered with cells with no free surface area, established cell line HRC is inoculated in the amount of $1—2 \times 10^7$ cells. The culture vessel used is a co-called Roux-bottle. The combined culture is then conducted by discharging the BME supplemented with 10% new born calf serum from the Roux-bottle, and then using fresh BME with no serum added. The medium is incubated at 37°C for 4 days followed by collection of the medium.

3) Dialysis and concentration

The collected medium is then placed in a Visking tube (a tube made of cellophane and conventionally used for dialysis) having its outer surface covered with polyethylene glycol, and is concentrated. Thereafter, dialysis is conducted with deionized 10 mM sodium bicarbonate.

4) Assay of inhibitor

Carcinoma cells to be used for assay include established cell lines originated from a human renal carcinoma (HRC) and from a carcinoma of the human oral cavity (KB). Media used for assay include the collected medium and the dialyzed medium which are respectively added in turn to a fresh medium supplemented with a 10% new born calf serum in a plastic dish. The carcinoma cells HRC and KB and normal human fibroblasts were incubated in the media and their proliferation measured.

5) Experimental results

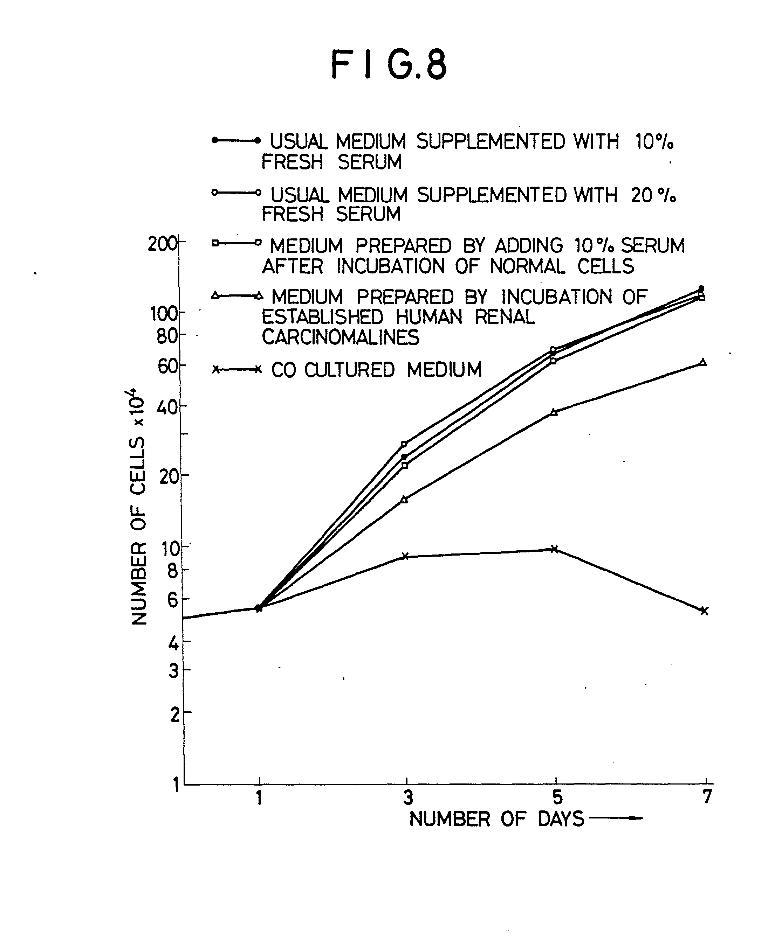

Fig. 8 shows proliferation curves indicating cell numbers of HRC with respect to the number of days of culture using various media, namely, a usual medium supplemented with 10% fresh serum, a usual medium supplemented with 20% fresh serum, a medium prepared by adding 10% serum after incubation of normal cells, a medium prepared by incubation of HRC, and a medium prepared by combined culture. The results indicate that only the incubation of the combined culture medium demonstrated inhibitive effects on proliferation and also demonstrates damage to the carcinoma cells.

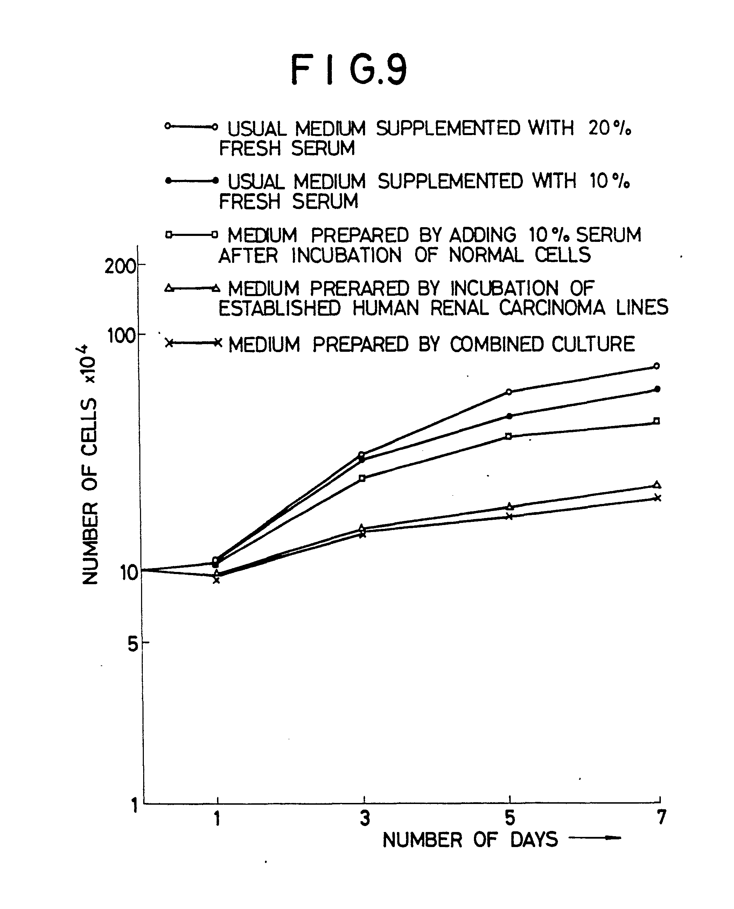

Fig. 9 shows the results of a similar experiment in which normal human fibroblasts were incubated in the various media used for the experiment of Fig. 8. In this case the co-cultured medium shows proliferation rather than cell number decrease.

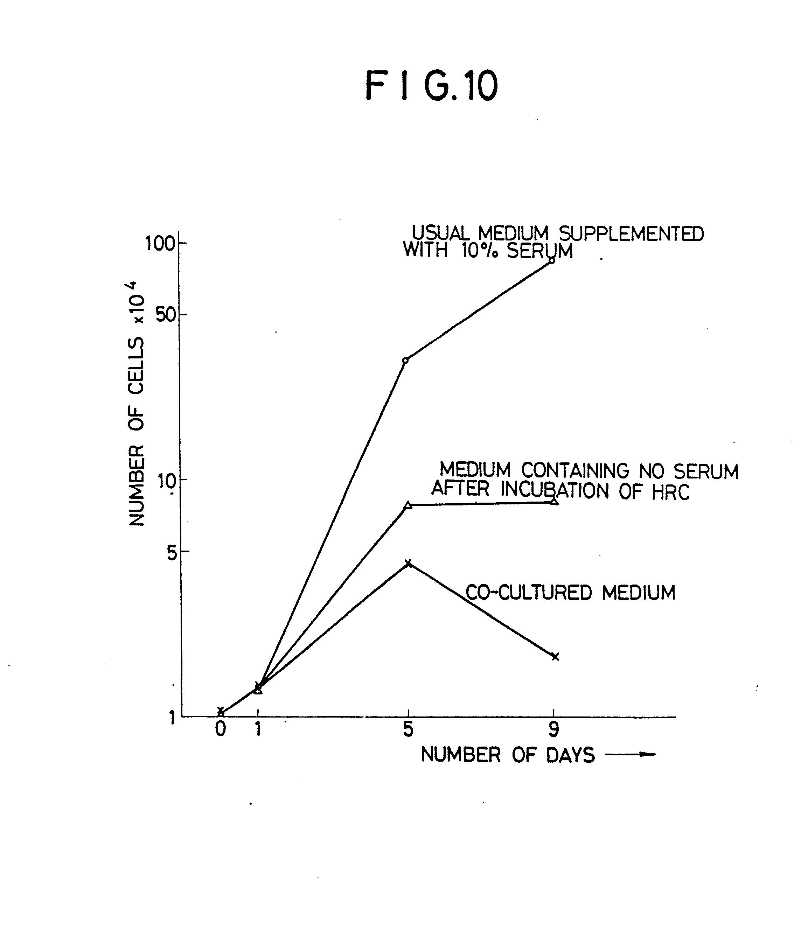

Fig. 10 shows the results of experiments conducted using a combined culture in a medium with no serum supplement and then incubating HRC in a medium prepared by adding 10% fresh serum to the co-cultured serum-less medium, and in a usual medium supplemented with a 10% serum, and in a medium containing no serum after incubation of HRC. The results indicate that combined culture in a medium containing no serum can give similar effects. This means that the extraction of an agent for inhibiting the proliferation of human tumour cells may be effected smoothly without the presence of serum interfering in any way with this proliferation.

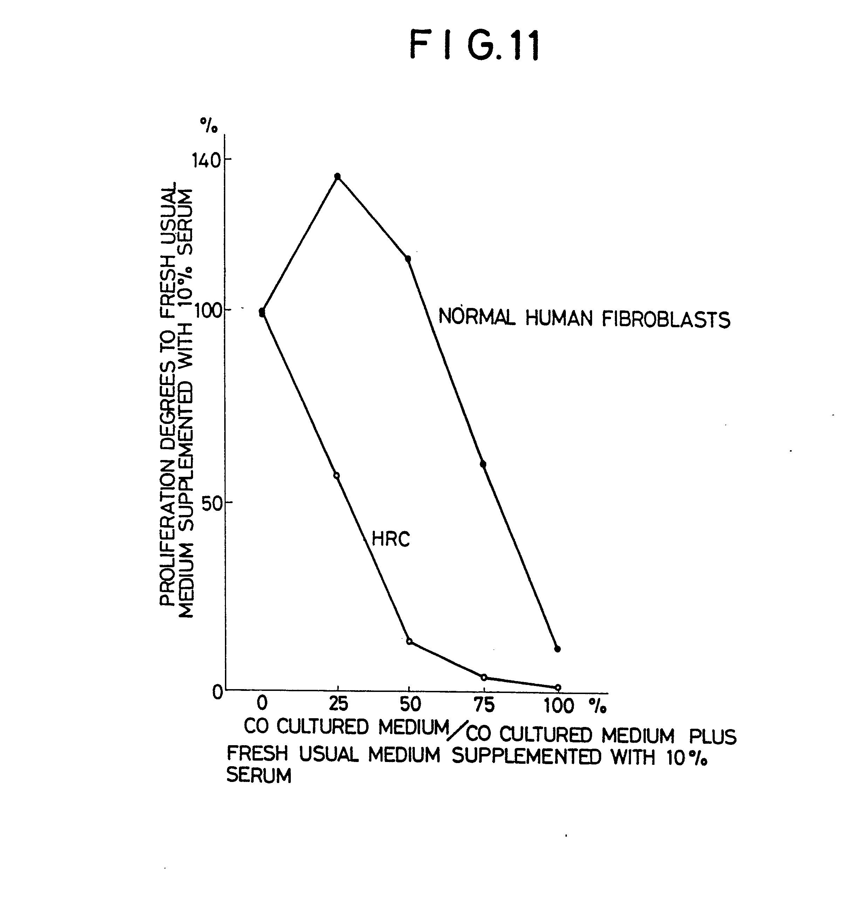

Fig. 11 indicates the degree of proliferation for normal human fibroblasts and HRC in media prepared by mixing the co-cultured media in varying percentage ratios thereof to the sum of the co-cultured medium and a fresh usual medium supplemented with 10% serum. The results show that normal cells proliferated in media of certain percentage ratios more than in a normal cell medium, and that the proliferation of tumour cells decreases in a logarithmic manner.

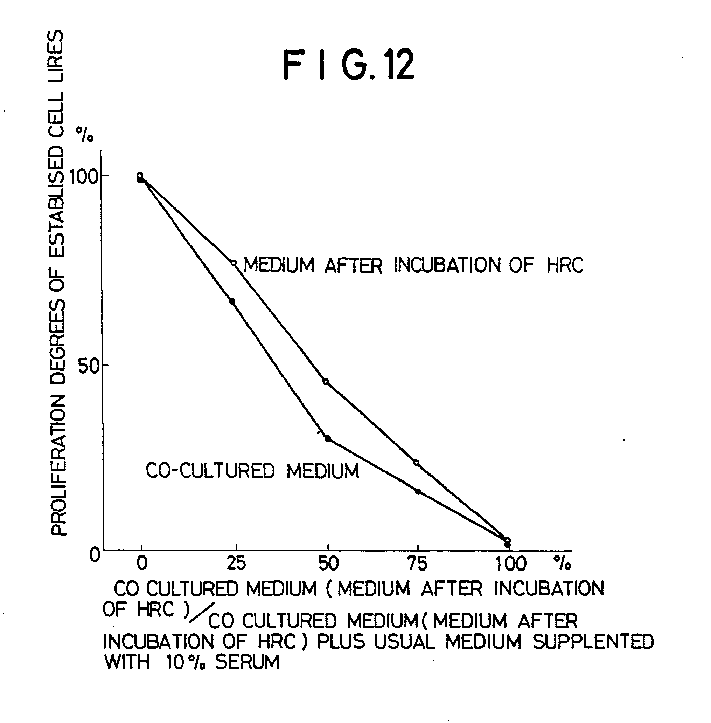

Fig. 12 indicates the degree of proliferation of KB in media comprising various ratios of either a co-cultured medium or a medium after incubation of HRC to the total amount of either of the said media plus a usual medium supplemented with 10% serum. The results indicate that, the higher the ratio of the co-cultured medium used, the higher the degree of inhibition. This implies that co-cultured medium contributes to the inhibition of the proliferation of tumour cells, and that inhibition is caused by an agent obtained therefrom.

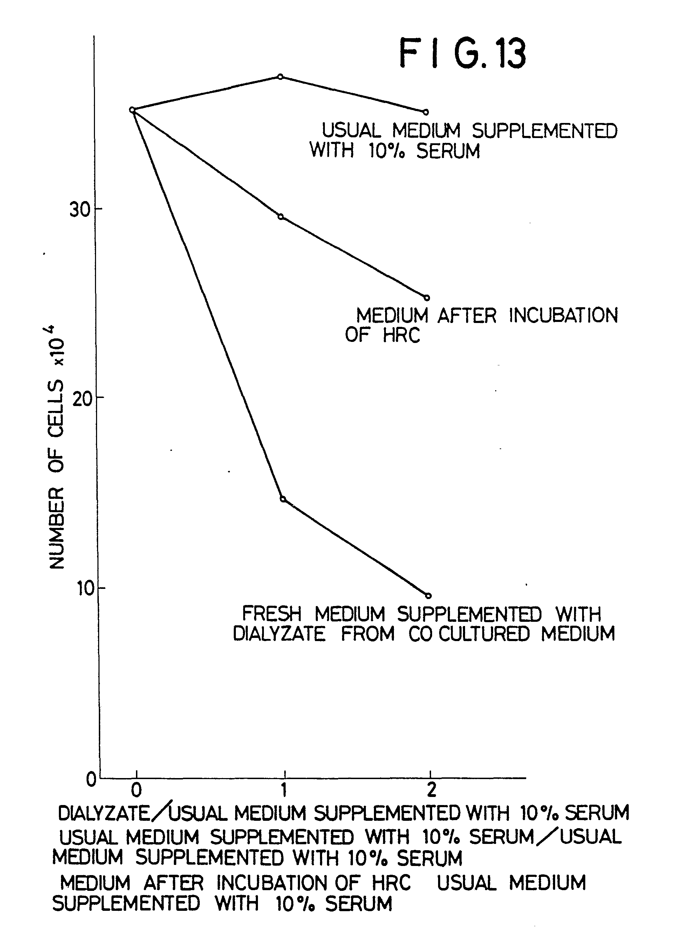

Fig. 13 indicates the variation in HRC cell numbers in a medium containing no serum in admixture with, respectively, a mixture of dialyzate of a co-cultured medium with a usual medium supplemented with 10% serum, with a medium after the incubation of HRC, and with a usual medium supplemented with 10% serum. The results indicate a remarkable inhibitive effect on cell proliferation in the medium supplemented with an extract from the co-cultured medium.

Even if human fibroblasts and tumour cells are not restricted to one type and are present in various forms, similar effects may be obtained. With varying ratios of normal cells to tumour cells, the strength of the effect may vary. From the results of the experiments set forth hereinabove, it is seen that tumour cells are preferably inoculated in amounts ranging from 10 to 20 million cells with respect to normal cells in amounts ranging from 7 to 10 million cells.

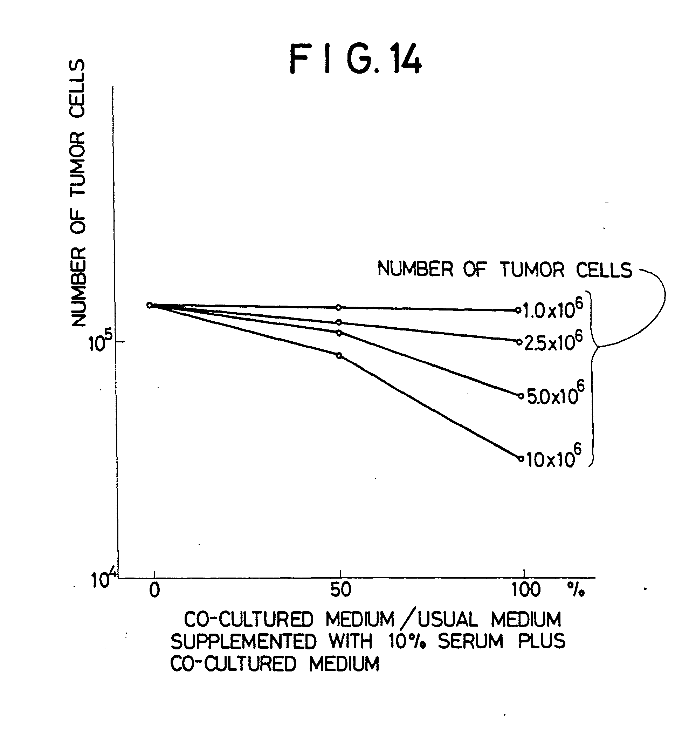

In Fig. 14, the abscissa indicates ratios of co-cultured medium to the sum of the usual medium supplemented with a 10% serum and medium for the combined culture, and the ordinate indicates the numbers of tumour cells after 5 days when the number of inoculated HRC carcinoma cells for the combined cultures and of HRC carcinoma cells of the same kind is varied. The results show that the larger the number of inoculated tumour cells or the higher the ratio, the smaller the number of proliferated tumour cells.

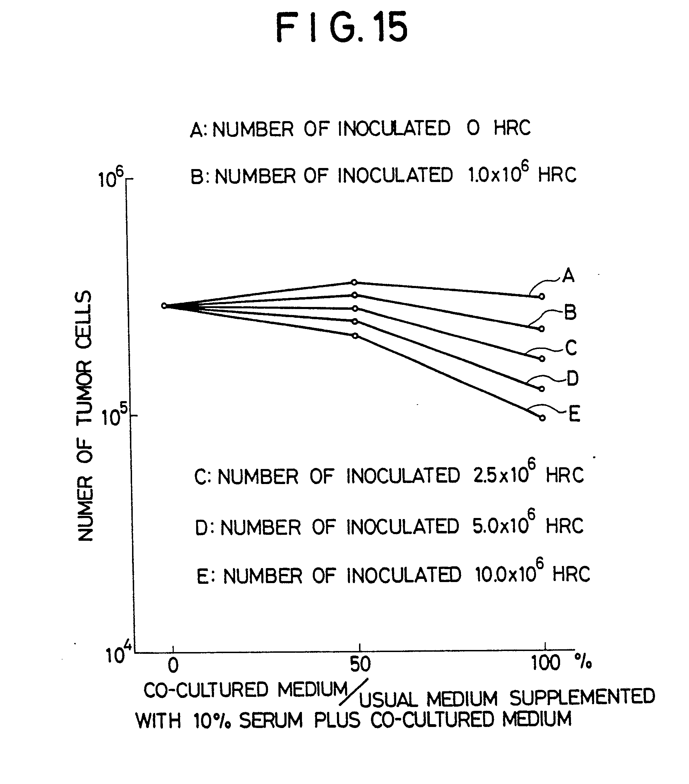

In Fig. 15, the abscissa indicates ratios of co-cultured medium to the sum of the usual medium supplemented with 10% serum and co-cultured medium, and the ordinate indicates the numbers of tumour cells after 5 days when the number of inoculated HRC carcinoma cells for the combined cultures and of HRC carcinoma cells of the same kind is varied. The results show that the larger the number of inoculated tumour cells or the higher the ratio, the smaller the number of proliferated tumour cells.

In Fig. 15, the abscissa indicates ratios of co-cultured medium to the sum of the usual medium supplemented with 10% serum and co-cultured medium, and the ordinate indicates the numbers of tumour cells after 5 days when the number of inoculated KB carcinoma cells, different from the HRC carcinoma cells used for the combined culture, is varied. Even when different carcinoma cells are used, a tendency similar to that shown in Fig. 14 is seen.

The growth medium employed need not be restricted to BME and may include other media. Concentrations of serum need not be restricted to 10% and may vary. Serum may include new born calf serum, cattle serum, calf serum and cattle embryo serum, and serum of other animals may also be used. Cultures may be of standing or settled culture type and of float culture type. Various culture vessels may be employed as appropriate.

**Claims**

1. An agent for inhibiting proliferation of malignant tumour cells, characterised in that said agent is obtained by the steps of: a) culturing in a medium supplemented with serum selected from new born calf serum, cattle serum, calf serum, cattle embryo serum and like serum derived from other species human malignant cells selected from human renal carcinoma cells, human gastric carcinoma cells, human lung carcinoma cells, carcinoma cells from the human oral cavity or human myogenic sarcoma cells; b) washing once to remove the serum; c) transferring the cultured human malignant tumour cells to a separation medium without serum supplement; d) incubating the thus transferred cells; and e) removing from said separation medium both said malignant tumour cells and molecules having a molecular weight of not less than 1000 daltons.

2. An agent according to Claim 1, further characterised in that said culture step is performed by conducting co-culture of said malignant tumour cells and normal fibroblasts.

3. An agent according to Claim 2, further characterised in that said normal fibroblasts are normal human diploid skin fibroblasts.

4. An agent according to any preceding claim, further characterised in that the said culture step is conducted in a growth medium comprising a synthetic medium supplemented with 5% or more new born calf serum.

5. An agent according to Claim 4, further characterised in that said synthetic medium is Basal Medium Eagle, and is supplemented with 10% new born calf serum.

6. An agent according to Claim 4, further characterised in that said synthetic meduim is "RPMI1646", and is supplemented with 5% new born calf serum.

7. An agent according to any preceding claim, further characterised in that said separation medium is a synthetic medium.

8. An agent according to Claim 7, further characterised in that said synthetic medium is Basal Medium Eagle.

9. An agent according to any preceding claim, further characterised in that removal of said malignant tumour cells from said separation medium is conducted using a molecular sieve capable of filtering out substances having a molecular weight of not less than 500 daltons.

10. A process for the production of an agent for inhibiting proliferation of malignant tumour cells, the process being characterised in that it comprises the steps of: a) culturing in a medium supplemented with serum selected from new born calf serum, cattle serum, calf serum, cattle embryo serum and like serum derived from other species human malignant cells selected from human renal carcinoma cells, human gastric carcinoma cells, human lung carcinoma cells, carcinoma cells from the human oral cavity or human myogenic sarcoma cells; b) washing once to remove the serum; c) transferring the cultured human malignant tumour cells to a separation medium without serum supplement; d) incubating the thus transferred cells; and e) removing from said separation medium both said malignant tumour cells and molecules having a molecular weight of not less than 1000 daltons.

**Patentansprüche**

1. Mittel zur Hemmung der Wucherung maligner Tumorzellen, dadurch gekennzeichnet, daß dieses Mittel erhalten wird durch folgende Schritte:

a) Kultivieren humaner maligner Zellen ausgewählt aus humanen Nierenkarzinomzellen, humanen Magenkarzinomzellen, humanen Lungenkarzinomzellen, Karzinomzellen der menschlichen Mundhöhle oder humanen Myogen-Sarkomzellen in einem Medium, dem ein Serum zugesetzt wurde ausgewählt aus Serum neugeborener Kälber, Rinderserum, Kalbsserum, Rinderembryoserum und vergleichbaren von anderen Spezies gewonnenen Seren; ·

b) einmaliges Waschen zur Entfernung des Serums;

c) Überführen der gezüchteten humanen, malignen Tumorzellen in ein Abtrennmedium ohne Serumzusatz;

d) Inkubieren der so überführten Zellen;

e) und Entfernen sowohl dieser malignen Tumorzellen als auch von Molekülen mit einem Molekulargewicht von nicht weniger als 1000 Dalton aus diesem Abtrennmedium.

2. Mittel gemäß Anspruch 1, dadurch gekennzeichnet, daß dieser Kulvitierungsschritt durch begleitende Co-Kultur dieser malignen Tumorzellen mit normalen Fibroblasten durchgeführt wird.

3. Mittel nach Anspruch 2, dadurch gekennzeichnet, daß diese normalen Fibroblasten normale humane diploide Hautfibroblasten sind.

4. Mittel gemäß einem der vorhergehenden Ansprüche, dadurch gekennzeichnet, daß dieser Kultivierungsschritt in einem Wachstumsmedium durchgeführt wird enthaltend ein synthetisches Medium, dem 5% oder mehr Serum von neugeborenen Kälbern zugesetzt sind.

5. Mittel nach Anspruch 4, dadurch gekennzeichnet, daß dieses synthetische Medium Basalmedium Eagle ist und daß diesem 10% Serum neugeborener Kälber zugesetzt wird.

6. Mittel nach Anspruch 4, dadurch gekennzeichnet, daß dieses synthetische Medium "RPMI1646" ist und diesem 5% Serum neugeborener Kälber zugesetzt wird.

7. Mittel gemäß einem der vorhergehenden Ansprüche, dadurch gekennzeichnet, daß das Abtrennmedium ein synthetisches Medium ist.

8. Mittel nach Anspruch 7, dadurch gekennzeichnet, daß das synthetische Medium Basalmedium Eagle ist.

9. Mittel nach einem der vorhergehenden Ansprüche, dadurch gekennzeichnet, daß die Entfernung der malignen Tumorzellen aus dem Abtrennmedium unter Verwendung eines Molekularsiebs durchgeführt wird, das in der Lage ist, Substanzen mit einem Molekulargewicht von nicht weniger als 500 Dalton herauszufiltern.

10. Verfahren zur Herstellung eines Mittels zur Hemmung der Wucherung maligner Tumorzellen, dadurch gekennzeichnet, daß es die Schritte umfaßt:

a) Kultivieren humaner maligner Zellen ausgewählt aus humanen Nierenkarzinomzellen, humanen Magenkarzinomzellen, humanen Lungenkarzinomzellen, Karzinomzellen der menschlichen Mundhöhle oder humanen Myogen-Sarkomzellen in einem Medium, dem ein Serum ausgewählt aus Serum neugeborener Kälber, Rinderserum, Kalbsserum, Rinderembryoserum und ähnlichen Seren anderer Spezies zugesetzt ist;

b) einmaliges Waschen zur Entfernung des Serums;

c) Überführen der kultivierten humanen malignen Tumorzellen in ein Abtrennmedium ohne Serumzusatz;

d) Inkubieren der so überführten Zellen; und

e) Entfernen sowohl der malignen Tumorzellen als auch von Molekülen mit einem Molekulargewicht von nicht weniger als 1000 Dalton aus diesem Abtrennmedium.

**Revendications**

1. Agent d'inhibition de la prolifération de cellules de tumeur maligne, caractérisé en ce que ledit agent est obtenu par les étapes de: a) mise en culture dans un milieu additionné de sérum choisi parmi un sérum de veau nouveau-né, un sérum de bétail, un sérum de veau, un sérum d'embryon de bétail et un sérum similaire dérivé d'autres cellules malignes d'espèce humaine choisies parmi des cellules carcinomales rénales humaines, des cellules carcinomales gastriques humaines, des cellules carcinomales de poumons humains, des cellules carcinomales de cavité orale humaine ou des cellules de sarcomes myogènes humains; b) lavage une fois pour éliminer le sérum; c) transfert des cellules de tumeur maligne humaines cultivées vers un milieu de séparation sans addition de sérum; d) incubation des cellules ainsi transférées; et e) élimination dudit milieu de séparation, à la fois des cellules de tumeur maligne et des molécules ayant un poids moléculaire non inférieur à 1.000 daltons.

2. Agent selon la revendication 1, caractérisé en outre en ce que ladite étape de culture est réalisée en conduisant une co-culture desdites cellules de tumeur maligne et de fibro-blastes normaux.

3. Agent selon la revendication 2, caractérisé en outre en ce que lesdits fibroblastes normaux sont des fibroblastes de peau de diploïde humain.

4. Agent selon l'une quelconque des revendications précédentes, caractérisé en outre en ce que ladite étape de culture est réalisée dans un milieu de croissance comprenant un milieu synthétique additionné d'au moins 5% de sérum de veau nouveau-né.

5. Agent selon la revendication 4, caractérisé en outre en ce que ledit milieu synthétique est le Basal Medium Eagle, et est additionné de 10% de sérum de veau nouveau-né.

6. Agent selon la revendication 4, caractérisé en outre en ce que ledit milieu synthétique est "RPMI1646", et est additionné de 5% de sérum de veau nouvea-né.

**0 102 801**

7. Agent selon l'une quelconque des revendications précédentes, caractérisé en outre en ce que ledit milieu de séparation est un milieu synthétique.

8. Agent selon la revendication 7, caractérisé en outre en ce que ledit milieu synthétique est le Basal Medium Eagle.

9. Agent selon l'une quelconque des revendications précédentes, caractérisé en outre en ce que l'élimination desdites cellules de tumeur maligne dudit milieu de séparation, est réalisée en utilisant un tamis moléculaire capable de séparer par filtration des substances ayant un poids moléculaire non inférieur à 500 daltons.

10. Procédé de production d'un agent d'inhibition de prolifération de cellules de tumeur maligne, le procédé étant caractérisé en ce qu'il comprend les étapes de: a) culture dans un milieu additionné de sérum choisi parmi un sérum de veau nouveau-né, un sérum de bétail, un sérum de veau, un sérum d'embryon de bétail et un sérum similaire dérivé d'autres cellules malignes d'espèce humaine choisies parmi des cellules carcinomales rénales humaines, des cellules carcinomales gastriques humaines, des cellules carcinomales de poumons humains, des cellules carcinomales de cavité orale humaine ou des cellules de sarcomes myogènes humains; b) lavaage une fois pour éliminer le sérum; c) transfert des cellules de tumeur maligne humaines cultivées vers un milieu de séparation sans addition de sérum; d) incubation des cellules ainsi tranférées; et e) élimination dudit milieu de séparation, à la fois des cellules de tumeur maligne et des molécules ayant un poids moléculaire non inférieur à 1.000 daltons.

0 102 801

# F I G. 1

# FIG. 2A

# FIG. 2B

# F I G.3

# F I G.4

# F I G.5

# F I G.6

# F I G.7

# F I G.8

●———● USUAL MEDIUM SUPPLEMENTED WITH 10% FRESH SERUM

○———○ USUAL MEDIUM SUPPLEMENTED WITH 20% FRESH SERUM

□———□ MEDIUM PREPARED BY ADDING 10% SERUM AFTER INCUBATION OF NORMAL CELLS

△———△ MEDIUM PREPARED BY INCUBATION OF ESTABLISHED HUMAN RENAL CARCINOMALINES

×———× CO CULTURED MEDIUM

NUMBER OF CELLS ×10⁴

NUMBER OF DAYS ——➤

# F I G.9

o——o USUAL MEDIUM SUPPLEMENTED WITH 20%
FRESH SERUM

•——• USUAL MEDIUM SUPPLEMENTED WITH 10%
FRESH SERUM

□——□ MEDIUM PREPARED BY ADDING 10% SERUM
AFTER INCUBATION OF NORMAL CELLS

△——△ MEDIUM PRERARED BY INCUBATION OF
ESTABLISHED HUMAN RENAL CARCINOMA LINES

×——× MEDIUM PREPARED BY COMBINED CULTURE

NUMBER OF CELLS $\times 10^4$

NUMBER OF DAYS ——

# F I G.10

# F I G.11

# F I G. 12

PROLIFERATION DEGREES OF ESTABLISED CELL LIRES

MEDIUM AFTER INCUBATION OF HRC

CO-CULTURED MEDIUM

CO CULTURED MEDIUM ( MEDIUM AFTER INCUBATION OF HRC )／CO CULTURED MEDIUM( MEDIUM AFTER INCUBATION OF HRC ) PLUS USUAL MEDIUM SUPPLENTED WITH 10 % SERUM

0 102 801

# F I G. 13

NUMBER OF CELLS $\times 10^4$

USUAL MEDIUM SUPPLEMENTED WITH 10% SERUM

MEDIUM AFTER INCUBATION OF HRC

FRESH MEDIUM SUPPLEMENTED WITH DIALYZATE FROM CO CULTURED MEDIUM

DIALYZATE/USUAL MEDIUM SUPPLEMENTED WITH 10% SERUM
USUAL MEDIUM SUPPLEMENTED WITH 10% SERUM/USUAL MEDIUM SUPPLEMENTED WITH 10% SERUM
MEDIUM AFTER INCUBATION OF HRC   USUAL MEDIUM SUPPLEMENTED WITH 10% SERUM

13

# F I G. 14

NUMBER OF TUMOR CELLS

NUMBER OF TUMOR CELLS

$1.0 \times 10^6$

$2.5 \times 10^6$

$5.0 \times 10^6$

$10 \times 10^6$

$10^5$

$10^4$

0  50  100  %

CO-CULTURED MEDIUM / USUAL MEDIUM
SUPPLEMENTED WITH 10% SERUM PLUS
CO-CULTURED MEDIUM

# F I G. 15

A: NUMBER OF INOCULATED 0 HRC

B: NUMBER OF INOCULATED $1.0 \times 10^6$ HRC

C: NUMBER OF INOCULATED $2.5 \times 10^6$ HRC

D: NUMBER OF INOCULATED $5.0 \times 10^6$ HRC

E: NUMBER OF INOCULATED $10.0 \times 10^6$ HRC

NUMER OF TUMOR CELLS

$10^6$

$10^5$

$10^4$

0    50    100  %

CO-CULTURED MEDIUM / USUAL MEDIUM SUPPLEMENTED WITH 10% SERUM PLUS CO-CULTURED MEDIUM